# FASCICULE DE BREVET EUROPEEN

(11) **EP 0 953 361 B1**
(45) Date de publication et mention de la délivrance du brevet: **13.02.2013**
(21) Numéro de dépôt: 99401004.9
(22) Date de dépôt: 23.04.1999
(51) Int. Cl.: A61M 1/02

(54) **Poche et ensemble de poches de filtration**
Filtrierbeutel, sowie Filtrierbeutelsatz
Fitration bag and filtration bag set

(30) Priorité: 27.04.1998 FR 9805266
(43) Date de publication de la demande: 03.11.1999
(73) Titulaire: Maco Pharma S.A., 59420 Mouvaux (FR)
(72) Inventeur: Dalle, Valéry, 59491 Villeneuve d'Asco (FR); Goudaliez, Francis, 59155 Paches Thumesnil (FR); Verpoort, Thierry, 59420 Mouvaux (FR)
(74) Mandataire: Herrou, Nathalie

(56) Documents cités:
- EP-A- 0 526 678
- EP-A- 0 645 151
- WO-A-98/19722
- DE-A- 3 521 735
- GB-A- 2 037 614
- US-A- 4 701 267
- US-A- 5 344 561
- US-A- 5 360 545
- US-A- 5 707 520

## Description

La présente invention se rapporte à une poche filtrante destinée à retenir par filtration les constituants cellulaires du plasma.

Elle se rapporte également à un ensemble de poches utilisant une telle poche filtrante.

Une poche filtrante destinée à la filtration du sang total ou des globules rouges est décrite dans EP 526678.

Depuis de nombreuses années, des dispositifs de prélèvement du sang ont été élaborés, notamment pour le recueil et la séparation de certains constituants du sang.

Ainsi, il existe des systèmes autorisant le prélèvement du sang total et la séparation des globules blancs à l'aide d'un filtre à déleucocyter, pour ne recueillir dans une poche de recueil que les globules rouges et le plasma.

Un tel système est par exemple décrit dans le documents US-A-5 707 520 qui révèle un milieu filtrant composé d'un matériau tridimensionnel présentant des pores ouverts et continus ayant une distribution de diamètres centrée entre 2 et 4 microns.

Les documents US-A-5 344 561, US-A-5 360 545 et US-A-4 701 267 décrivent également un dispositif pour déleucocyter une préparation sanguine dans lequel le milieu filtrant est de préférence fibreux, par exemple sous la forme d'un non-tissé. De tels milieux filtrants présentent également une large distribution de taille de pores.

De tels systèmes de filtration, parfois qualifiés de filtres en profondeur, ne permettent pas d'obtenir un plasma exempt de constituant cellulaire car il ne présentent pas un seuil de coupure défini du fait de la distribution relativement large de la taille des pores.

Le plasma sanguin comprend, en plus de l'eau, des constituants protéiniques, telles que albumine, facteurs de coagulation, immuno-globulines.

Ces constituants possèdent des propriétés importantes et peuvent entrer dans la préparation et la composition de produits pharmaceutiques.

Ils permettent également de recueillir des renseignements importants sur l'état de santé du donneur.

Toutefois, le plasma obtenu après séparation par centrifugation des constituants cellulaires, notamment globules rouges et globules blancs, présente encore un taux non négligeable en constituants cellulaires résiduels.

L'invention a pour but de remédier à ce problème, en proposant une poche filtrante permettant la séparation par filtration des constituants cellulaires du plasma, avec une efficacité élevée, telle que définie dans les revendications.

A cet effet, la poche filtrante de l'invention, destinée à retenir par filtration les constituants cellulaires du plasma, comprend une enveloppe extérieure munie d'au moins un orifice d'entrée et d'au moins un orifice de sortie.

L'enveloppe extérieure renferme un milieu filtrant qui délimite deux compartiments, respectivement d'entrée et de sortie de la poche filtrante, le milieu filtrant de la poche filtrante comportant au moins une couche d'un matériau hydrophile sous forme d'une membrane poreuse dont les pores sont calibrés à une taille apte à empêcher le passage des constituants cellulaires, à savoir n'étant pas supérieure à 3 microns.

Par exemple, la membrane poreuse présente une taille de pores calibrée à 0,65 micron de sorte à fournir un seuil de coupure défini. Cette membrane permet alors de retenir sur sa surface amont les constituants cellulaires du plasma et notamment les globules rouges. Un tel milieu filtrant est parfois qualifié de filtre en surface ou filtre écran.

Ainsi, le milieu filtrant stoppe les constituants cellulaires du plasma, notamment les globules rouges ou érythrocytes, les globules blancs ou leucocytes ainsi que les plaquettes sanguines.

Différents matériaux peuvent être utilisés pour la réalisation du milieu filtrant.

Les caractéristiques de porosité, dues à la présence de pores dans le matériau constituant le milieu filtrant, et le caractère hydrophile permettant la mouillabilité du milieu filtrant lors du passage du plasma, peuvent être obtenues par le choix de matériaux hydrophiles naturellement ou de matériaux, notamment à base de matière plastique, rendus hydrophiles par un traitement particulier.

Des matériaux généralement hydrophiles naturellement et utilisables dans le cadre de l'invention sont, par exemple, les produits à base de cellulose, par exemple l'acétate de cellulose et ses dérivés.

D'autres matériaux utilisables pour la réalisation du milieu filtrant sont par exemple les matières plastiques, notamment les polymères et/ou copolymères à base de polypropylène, de polyester, de polyamide, de polyéthylène haute ou basse densité, de polyuréthane.

Un exemple particulier de polymère utilisable est le fluorure de polyvinylidène.

Ces produits polymériques ne sont généralement pas hydrophiles naturellement et doivent être traités par des méthodes physiques ou chimiques, pour leur conférer lesdites propriétés hydrophiles.

Ces traitements consistent par exemple dans le greffage de substituants hydrophiles, par exemple des groupements de type hydroxyle ou carboxylique, sur le polymère, selon des méthodes connues.

De tels polymères rendues hydrophiles par traitement physique ou chimique sont disponibles sur le marché.

Selon l'invention, la poche filtrante de l'invention comporte de plus un pré-filtre disposé dans le compartiment d'entrée de la poche filtrante et contre le milieu filtrant.

Ce pré-filtre comporte au moins une couche d'un matériau poreux, la taille des pores du pré-filtre étant comprise entre environ 8 et environ 100 microns.

Le pré-filtre a essentiellement pour fonction d'arrêter la fibrine, les grosses particules, les agrégats plaquettaires et ainsi de diminuer les risques de colmatage du milieu filtrant.

Selon un autre mode de réalisation, la poche filtrante comporte de plus un post-filtre disposé dans le compartiment de sortie de la poche filtrante et contre le milieu filtrant, le post-filtre comportant au moins une couche d'un matériau poreux, la taille des pores du post-filtre étant comprise entre environ 8 et environ 100 microns.

Le post-filtre joue essentiellement un rôle de protection du milieu filtrant et ne filtre pas, à proprement parler, le plasma, qui est déjà passé à travers le milieu filtrant et éventuellement le pré-filtre.

Les matériaux utilisables pour la réalisation du post-filtre et du pré-filtre peuvent être choisis notamment parmi les matériaux poreux à base de matière plastique, notamment les polymères et/ou copolymères à base de polyéthylène haute ou basse densité, notamment le téréphtalate de polyéthylène, les polymères à base de polypropylène, de polyuréthane, de polyamide, par exemple le NYLON, par exemple sous forme d'un non-tissé.

Selon l'invention, le milieu filtrant comporte une seule couche de matériau poreux hydrophile.

Le pré-filtre et/ou le post-filtre peuvent comporter plusieurs couches de matériau poreux, de nature identique ou différente entre eux, de taille de pores identique ou différente entre eux.

Généralement, lorsque le pré-filtre comporte plusieurs couches de matériau poreux, les couches de plus grande porosité sont disposées de façon à ce que le plasma à filtrer entrant dans le compartiment d'entrée passe en premier à travers ces couches de plus grande porosité, puis ensuite à travers les couches de plus faible porosité avant d'atteindre le milieu filtrant.

Selon un mode d'exécution, le pré-filtre comporte cinq couches de matériau poreux.

Selon ce mode de réalisation, le pré-filtre comporte une couche de téréphtalate de polyéthylène d'une taille de pores moyenne d'environ 40 microns, disposée du côté de l'enveloppe extérieure et quatre couches de polypropylène d'une taille de pores moyenne d'environ 10 microns, disposées du côté du milieu filtrant.

Généralement, le milieu filtrant possède une épaisseur supérieure à environ 10 microns, notamment d'environ 100 microns.

Il va de soi cependant que l'épaisseur du milieu filtrant peut varier dans une large mesure, une faible épaisseur contribuant par exemple à accélérer le processus de filtration mais rendant le milieu filtrant plus fragile, une épaisseur plus importante améliorant la solidité du milieu filtrant mais présentant une incidence négative sur la vitesse de filtration.

Selon d'autres caractéristiques, le milieu filtrant possède une surface comprise entre environ 10 et environ 150 cm².

Selon une forme de réalisation, le milieu filtrant possède une surface de 57 cm².

La poche filtrante de l'invention peut se présenter sous la forme d'une poche rigide, par exemple en utilisant une enveloppe extérieure rigide.

En variante, la poche filtrante de l'invention peut se présenter sous la forme d'une poche souple, par exemple en utilisant une enveloppe extérieure souple.

L'invention vise également à protéger un ensemble de poches pour la séparation par filtration des constituants cellulaires du plasma.

L'ensemble de poches de l'invention comprend au moins une poche principale de prélèvement, reliée par l'intermédiaire d'une tubulure à une poche filtrante telle qu'il vient d'être décrit, la poche filtrante étant elle-même reliée, par l'intermédiaire d'une tubulure, à une poche secondaire de recueil.

Selon un mode de réalisation, la poche secondaire est reliée par l'intermédiaire de tubulures à une ou plusieurs poches satellites.

L'ensemble des poches de prélèvement, de recueil, filtrante, ainsi que les tubulures peuvent être souples et sécables. Il est également envisageable que les tubulures soient reliées aux différentes poches de façon fixe, par exemple par soudage.

L'ensemble de poches de l'invention peut ainsi être stérilisé et utilisé dans des opérations de prélèvement en circuit fermé.

L'invention sera mieux comprise dans la description qui suit, faite en référence aux figures annexées.
La figure 1 représente, vu de côté et en coupe longitudinale, un mode de réalisation de la poche filtrante.
La figure 2 représente, en vue de face et en coupe longitudinale partielle, la mise en place du milieu filtrant sur un cadre souple.
La figure 3 représente, vu de dessus et en coupe transversale, un mode de réalisation de la poche filtrante, montrant en particulier l'assemblage du cadre renfermant le filtre dans l'enveloppe extérieure.
La figure 4 représente, vu de face et en coupe partielle longitudinale, la poche filtrante de la figure 3 sur laquelle apparaissent les joncs d'écartement.
La figure 5 représente une vue schématique de face d'un mode de réalisation de l'ensemble de poches.
La figure 6 représente une vue schématique de face d'un deuxième mode de réalisation de l'ensemble de poches.

En se référant maintenant aux figures, la poche filtrante souple 1 de l'invention comporte une enveloppe extérieure souple formée de l'assemblage de deux feuilles de matière plastique 2 et 3 assemblées mutuellement sur leur périphérie.

Cette enveloppe extérieure renferme un filtre désigné de façon générale par la référence 4.

Le filtre 4 est maintenu dans un cadre support 5 souple et étanche et délimite deux compartiments, respectivement d'entrée 6 et de sortie 7 de la poche filtrante 1.

Le compartiment d'entrée 6 communique avec l'extérieur de la poche 1 par l'intermédiaire d'une tubulure d'entrée 8 qui sert au remplissage de la poche filtrante 1.

Le compartiment de sortie 7 communique avec l'extérieur de la poche 1 par l'intermédiaire d'une tubulure de sortie 9 qui assure l'évacuation du filtrat.

Selon une forme de réalisation, les tubulures d'entrée 8 et de sortie 9 sont souples.

Le filtre 4 comporte un pré-filtre 10 disposé dans le compartiment d'entrée 6, faisant face à l'enveloppe extérieure 2 et contre le milieu filtrant 11.

Le filtre 4 comporte également, dans le compartiment de sortie 7, faisant face à l'enveloppe extérieure 3 et contre le milieu filtrant 11, un post-filtre 12.

Il va de soi cependant que le filtre 4 peut ne comporter que le milieu filtrant 11 et le pré-filtre 10.

Un premier niveau d'étanchéité de la poche filtrante 1 est assuré entre le filtre 4 et le cadre souple 5 où il n'y a aucun passage de tubulure.

Un second niveau d'étanchéité est assuré à la périphérie de la poche filtrante 1 où les deux feuilles extérieures 2 et 3, la périphérie du cadre souple 5 et le passage des tubulures souples d'entrée 8 et de sortie 9 sont jointes.

Ce second niveau d'étanchéité peut être assuré par les techniques connues de liaison de matériaux plastiques, par exemple par soudage à haute fréquence.

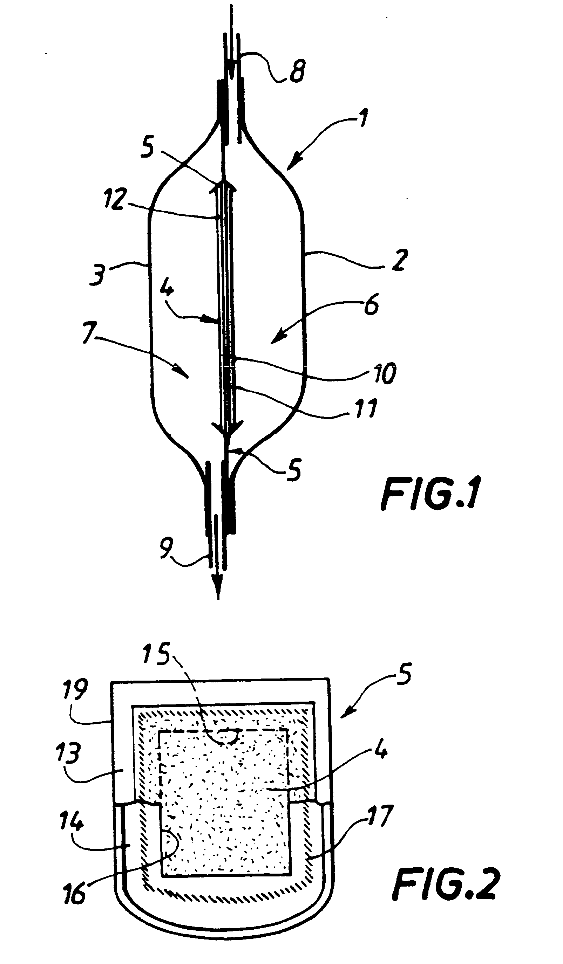

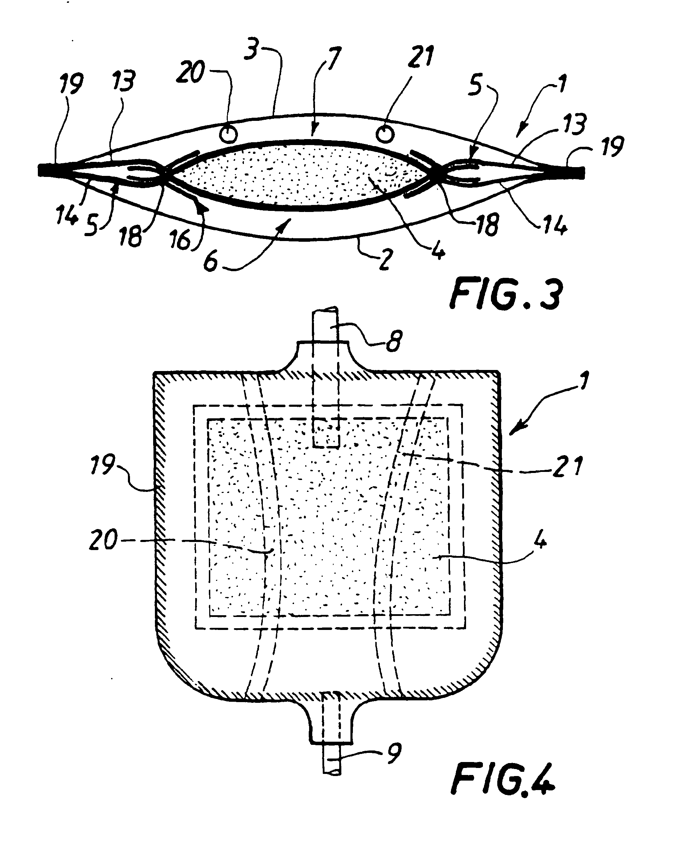

En se référant maintenant aux figures 2 à 4, l'assemblage de la poche filtrante 1 est décrit. Sur ces figures, pour plus de clarté, le filtre est représenté de façon générale par la référence 4, sans que soient représentés le pré-filtre 10 et le post-filtre 12.

Le filtre 4 tel que représenté sur les figures 2 à 4 doit cependant s'entendre comme pouvant comporter le milieu filtrant 11 et le pré-filtre 10 et/ou le post-filtre 12.

Le cadre souple 5 est formé par un assemblage de deux feuilles, par exemple plastifiées, 13 et 14 entre lesquelles est placé le filtre 4.

Ces deux feuilles 13 et 14 sont ajourées dans leur partie centrale et comportent chacune au moins une ouverture 15 et 16 permettant le passage du liquide à filtrer.

Les deux feuilles 13 et 14 sont fixées entre elles de préférence au niveau de la périphérie du filtre 4, par exemple par un cordon de soudure 17, réalisé à travers le filtre 4, assurant à la fois la fixation du filtre 4 mais également l'étanchéité du système.

La soudure des feuilles 13 et 14 à travers le filtre 4 provoque une compression 18, formant un cordon étanche autour du filtre 4.

La périphérie 19 du cadre souple 5 est soudée également avec les feuilles extérieures 2 et 3 formant l'enveloppe de la poche filtrante 1, mutuellement sur tout leur pourtour et au niveau de leur périphérie, assurant ainsi l'étanchéité du système.

Pour éviter que le filtre 4 ne se colle contre l'enveloppe extérieure 3, et gène ainsi l'écoulement du filtrat dans le compartiment de sortie 7, deux joncs d'écartement 20 et 21 sont placés à l'intérieur du compartiment de sortie 7, entre le filtre 4 et l'enveloppe extérieure 3.

Ces deux joncs 20 et 21 dégagent le compartiment de sortie 7 du filtre 4 et évitent ainsi que le filtre 4 ne se plaque contre la paroi intérieure de la feuille extérieure 3.

Les joncs 20 et 21 peuvent être réalisés à partir de tubulures souples soudées par exemple au niveau de la paroi intérieure de la feuille 3 de l'enveloppe extérieure, par exemple au niveau de la soudure périphérique 19 de la poche filtrante 1.

Il va de soi que le nombre de joncs d'écartement peut varier, en fonction par exemple des dimensions de la poche filtrante 1.

Par exemple, il est envisageable de prévoir un jonc d'écartement unique replié de façon à former une boucle à l'intérieur du compartiment de sortie.

De préférence, des joncs 20 et 21 souples sont utilisés, pour ne pas gêner les possibilités de pliage de la poche filtrante 1.

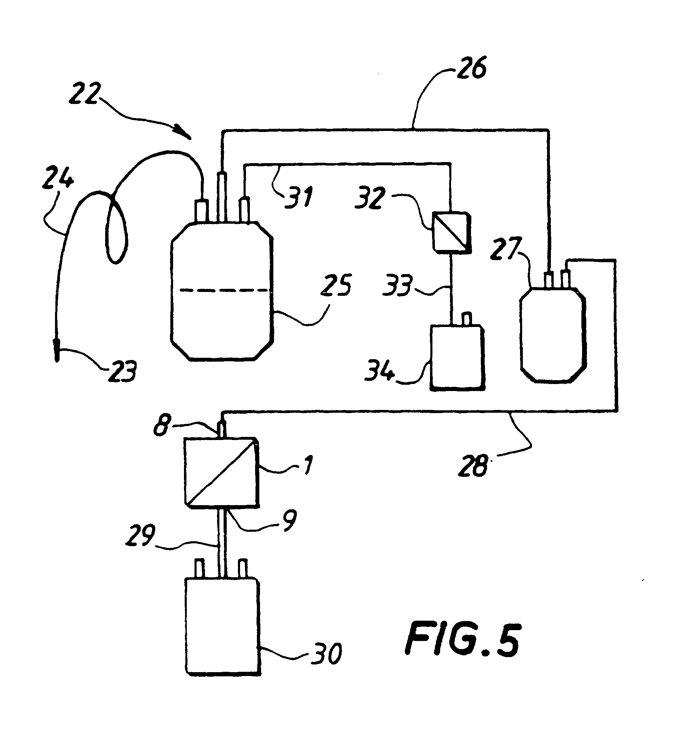

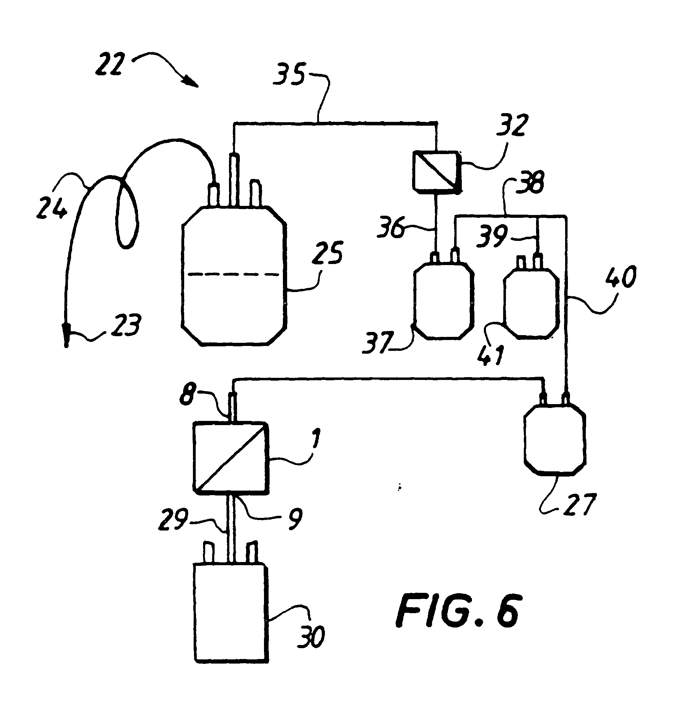

En se référant maintenant aux figures 5 et 6, un ensemble de poches 22 est représenté, permettant la séparation par filtration des constituants cellulaires du plasma.

L'ensemble de poches 22 comprend une poche de recueil 27 recevant le plasma débarrassé par centrifugation d'une partie de ses constituants cellulaires.

La poche 27 est reliée, au niveau de l'un de ses orifices de sortie, à l'une des extrémités d'une tubulure 28 communiquant par son autre extrémité avec l'orifice d'entrée 8 de la poche filtrante 1 de l'invention.

L'orifice de sortie 9 de la poche filtrante 1 est relié à l'une des extrémités d'une tubulure 29, l'autre extrémité de cette tubulure 29 étant reliée à une poche de recueil 30, destinée à récolter le plasma filtré et débarrassé de ses constituants cellulaires.

Selon une première forme de réalisation (figure 5), l'ensemble de poches 22 comporte, en amont de la poche de recueil 27, une aiguille de prélèvement 23 destinée au prélèvement du sang d'un donneur, reliée à l'une des extrémités d'une tubulure 24, elle-même reliée par son autre extrémité à l'orifice d'entrée d'une poche de prélèvement 25.

La poche de prélèvement 25 est elle-même reliée, au niveau de l'un de ses orifices de sortie, à l'une des extrémités d'une tubulure 26 communiquant, par son autre extrémité, avec l'orifice d'entrée de la poche de recueil 27.

La poche de prélèvement 25 est reliée également, au niveau d'un autre de ses orifices de sortie, à l'une des extrémités d'une tubulure 31 communiquant par son autre extrémité avec l'orifice d'entrée d'un filtre à déleucocyter 32.

L'orifice de sortie du filtre à déleucocyter 32 communique lui-même par l'intermédiaire d'une tubulure 33 avec une poche de recueil 34.

Selon ce mode de réalisation, le sang est prélevé d'un donneur et est recueilli dans la poche de prélèvement 25. Après centrifugation, le plasma, contenant encore des constituants cellulaires résiduels, est envoyé dans la poche de recueil 27, tandis que le concentré globulaire reste dans la poche de prélèvement 25.

Après soudure de la tubulure 26 et éventuellement séparation d'avec la poche de prélèvement 25, le plasma est filtré par passage dans la poche filtrante 1 et est recueilli dans la poche de recueil 30.

Le concentré globulaire restant dans la poche de prélèvement 25 peut être ensuite passé à travers le filtre à déleucocyter 32, et le filtrat contenant essentiellement les globules rouges est recueilli dans la poche 34.

Selon une autre variante de réalisation (figure 6), l'ensemble de poches 22 comporte, en amont de la poche de recueil 27, une aiguille de prélèvement 23 destinée au prélèvement du sang d'un donneur, reliée à l'une des extrémités d'une tubulure 24, elle-même reliée par son autre extrémité à l'orifice d'entrée d'une poche de prélèvement 25.

La poche de prélèvement 25 est elle-même reliée, au niveau de l'un de ses orifices de sortie, à l'une des extrémités d'une tubulure 35 communiquant par son autre extrémité avec l'orifice d'entrée d'un filtre à déleucocyter 32.

L'orifice de sortie du filtre 32 est relié à l'une des extrémités d'une tubulure 36 communiquant par son autre extrémité avec l'orifice d'entrée d'une poche 37.

La poche 37 communique au niveau de son orifice de sortie avec une tubulure 38 se divisant en deux tubulures 39 et 40 communiquant avec les orifices d'entrée de deux poches 41 et 27 respectivement.

La poche 41 contient une solution de conservation, par exemple à base de SAGM.

Selon ce mode de réalisation, le sang est prélevé d'un donneur par l'aiguille 23 et arrive dans la poche de prélèvement 25.

Le sang est ensuite filtré par passage à travers le filtre à déleucoyter 32 et le filtrat est recueilli dans la poche 37.

Après centrifugation de la poche 37, et éventuellement soudure de la tubulure 36 et séparation d'avec la poche de prélèvement 25, le plasma est envoyé dans la poche de recueil 27, par l'intermédiaire de la tubulure 40, en obturant la tubulure 39 de façon classique.

Le plasma passe ensuite à travers la poche filtrante 1 et est recueilli, débarrassé de ses constituants cellulaires dans la poche de recueil 30.

La solution de conservation présente dans la poche 41 est ensuite passée dans la poche 37 contenant le sang débarrassé des leucocytes, par l'intermédiaire des tubulures 39 et 38.

Selon un mode de réalisation, l'ensemble de poches, les tubulures sont souples et les tubulures sont fixées de façon fixe aux poches, par exemple par soudage ou collage.

Egalement, il peut être prévu que les tubulures soient sécables, pour permettre la désolidarisation d'une partie de l'ensemble de poches.

## Revendications

1. Poche filtrante (1), destinée à retenir par filtration les constituants cellulaires du plasma, comprenant une enveloppe extérieure (2, 3) munie d'au moins un orifice d'entrée (8) et d'au moins un orifice de sortie (9), l'enveloppe renfermant un milieu filtrant (11) qui délimite deux compartiments respectivement d'entrée (6) et de sortie (7) de la poche filtrante (1) et un pré-filtre (10) disposé dans le compartiment d'entrée (6) et contre le milieu filtrant (11), ledit pré-filtre (10) comportant au moins une couche d'un matériau poreux, la taille des pores du pré-filtre (10) étant comprise entre environ 8 et environ 100 microns, **caractérisée en ce que** le milieu filtrant (11) de la poche filtrante (1) comporte une seule couche d'un matériau hydrophile sous forme d'une membrane poreuse dont les pores sont calibrés à une taille apte à empêcher le passage des constituants cellulaires, à savoir n'étant pas supérieure à 3 microns.

2. Poche filtrante (1) selon la revendication 1, **caractérisée en ce que** le compartiment d'entrée (6) communique avec l'extérieur de la poche (1) par l'intermédiaire d'une tubulure d'entrée (8).

3. Poche filtrante (1) selon la revendication 1 ou 2, **caractérisée en ce que** le compartiment de sortie (7) communique avec l'extérieur de la poche (1) par l'intermédiaire d'une tubulure de sortie (9).

4. Poche filtrante (1) selon la revendication 2 ou 3, **caractérisée en ce que** les tubulures d'entrée (8) et de sortie (9) sont souples.

5. Poche filtrante (1) selon l'une quelconque des revendications 1 à 4, **caractérisée en ce qu'**elle comporte un post-filtre (12) disposé dans le compartiment de sortie (7) de la poche filtrante (1) et contre le milieu filtrant (11), le post-filtre (12) comportant au moins une couche d'un matériau poreux, la taille des pores du post-filtre (12) étant comprise entre environ 8 et environ 100 microns.

6. Poche filtrante (1) selon l'une quelconque des revendications 1 à 5, **caractérisée en ce que** le matériau poreux hydrophile du milieu filtrant (11) est choisi notamment parmi les matériaux hydrophiles naturellement ou les matériaux, notamment à base de matière plastique, rendus hydrophiles.

7. Poche filtrante (1) selon la revendication 6, **caractérisée en ce que** le matériau hydrophile poreux est choisi notamment parmi les polymères et/ou copolymères à base de polypropylène, de polyester, de polyamide, de polyéthylène haute ou basse densité, de polyuréthane, le fluorure de polyvinylidène, les produits à base de cellulose, notamment l'acétate de cellulose et ses dérivés.

8. Poche filtrante (1) selon l'une quelconque des revendications 1 à 7, **caractérisée en ce que** le matériau poreux utilisé pour la réalisation du post-filtre (12) et du pré-filtre (10) est choisi notamment parmi les matériaux poreux à base de matière plastique, notamment les polymères et/ou copolymères à base de polyéthylène haute ou basse densité, notamment le téréphtalate de polyéthylène, les polymères à base de polypropylène, de polyamide, par exemple le NYLON, par exemple sous la forme d'un non-tissé.

9. Poche filtrante (1) selon l'une quelconque des revendications 1 à 8, **caractérisée en ce que** le pré-filtre (10) et/ou le post-filtre (12) comporte plusieurs couches de matériau poreux, de nature identique ou différente entre eux, de taille de pores identique ou différente entre eux.

10. Poche filtrante (1) selon l'une quelconque des revendications 1 à 9, **caractérisée en ce que** le pré-filtre (10) comporte plusieurs couches de matériau poreux, les couches de plus grande porosité étant disposées de façon à ce que le plasma à filtrer entrant dans le compartiment d'entrée (6) passe en premier à travers ces couches de plus grande porosité, puis ensuite à travers les couches de plus faible porosité avant d'atteindre le milieu filtrant (11).

11. Poche filtrante (1) selon la revendication 10, **caractérisée en ce que** le pré-filtre (10) comporte une couche de téréphtalate de polyéthylène d'une taille de pores moyenne d'environ 40 microns, disposée du côté de l'enveloppe extérieure (2) et quatre couches de polypropylène d'une taille de pores moyenne d'environ 10 microns, disposées du côté du milieu filtrant (11).

12. Poche filtrante (1) selon l'une quelconque des revendications 1 à 11, **caractérisée en ce que** le milieu filtrant (11) possède une épaisseur supérieure à environ 10 microns, notamment d'environ 100 microns.

13. Poche filtrante (1) selon l'une quelconque des revendications 1 à 12, **caractérisée en ce qu'**elle possède une surface comprise entre environ 10 cm² et environ 150 cm², par exemple 57 cm².

14. Poche filtrante (1) selon l'une quelconque des revendications 1 à 13, **caractérisée en ce que** l'enveloppe extérieure (2, 3) est rigide.

15. Poche filtrante (1) selon l'une quelconque des revendications 1 à 13, **caractérisée en ce que** l'enveloppe extérieure (2, 3) est souple.

16. Poche filtrante (1) selon la revendication 15, **caractérisée en ce que** l'enveloppe extérieure souple est formée de deux feuilles (2, 3) de matière plastique souple assemblées sur leur périphérie, le milieu filtrant (11) et/ou le pré-filtre (10) et/ou le post-filtre (12) étant maintenus dans un cadre souple et étanche (5) délimitant avec le milieu filtrant (11) et/ou le pré-filtre (10) et/ou le post-filtre (12) les compartiments d'entrée (6) et de sortie (7) de la poche filtrante (1).

17. Poche filtrante (1) selon la revendication 15 ou 16, **caractérisée en ce que** le cadre souple (5) est formé de deux feuilles souples (13, 14) ajourées entre elles entre lesquelles le milieu filtrant (11) et/ou le pré-filtre (10) et/ou le post-filtre (12) sont placés.

18. Poche filtrante (1) selon l'une quelconque des revendications 15 à 17, **caractérisée en ce que** les feuilles (13, 14) formant le cadre souple (5) sont fixées entre elles au niveau de la périphérie du milieu filtrant (11) et/ou du pré-filtre (10) et/ou du post-filtre (12) et également avec les feuilles (2, 3) formant l'enveloppe extérieure, au niveau de la périphérie de l'enveloppe extérieure de la poche filtrante (1).

19. Poche filtrante (1) selon l'une quelconque des revendications 15 à 18, **caractérisée en ce que** la fixation des feuilles (13, 14) formant le cadre souple (5) est un cordon de soudure (17) réalisé à travers le milieu filtrant (11) et/ou le pré-filtre (10) et/ou le post-filtre (12).

20. Poche filtrante (1) selon l'une quelconque des revendications 15 à 19, **caractérisée en ce que** le compartiment de sortie (7) est dégagé du milieu filtrant (11) et/ou du post-filtre (12) par la présence d'un ou plusieurs joncs d'écartement (20, 21), disposés entre le milieu filtrant (11) et/ou le post-filtre (12) et l'enveloppe extérieure souple, à l'intérieur du compartiment de sortie (7).

21. Poche filtrante (1) selon la revendication 20, **caractérisée en ce que** le ou les joncs d'écartement (20, 21) sont réalisés à partir de tubulures souples soudées par exemple au niveau de la paroi intérieure de la feuille (3) de l'enveloppe extérieure.

22. Ensemble de poches (22) pour la séparation par filtration des constituants cellulaires du plasma, comprenant une poche de recueil (27) recevant le plasma débarrassé par centrifugation d'une partie de ses constituants cellulaires, la poche de recueil (27) étant reliée, au niveau de l'un de ses orifices de sortie, à l'une des extrémités d'une tubulure (28) communiquant par son autre extrémité avec l'orifice d'entrée (8) d'une poche filtrante (1) selon l'une quelconque des revendications 1 à 21, l'orifice de sortie (9) de la poche filtrante (1) étant relié à l'une des extrémités d'une tubulure (29), l'autre extrémité de cette tubulure (29) étant reliée à une poche de recueil (30), destinée à récolter le plasma filtré et débarrassé de ses constituants cellulaires.

23. Ensemble de poches (22) selon la revendication 22, **caractérisé en ce qu'**il comporte, en amont de la poche de recueil (27), une aiguille de prélèvement (23) destinée au prélèvement du sang d'un donneur, reliée à l'une des extrémités d'une tubulure (24), elle-même reliée par son autre extrémité à l'orifice d'entrée d'une poche de prélèvement (25), la poche de prélèvement (25) étant elle-même reliée, au niveau de l'un de ses orifices de sortie, à l'une des extrémités d'une tubulure (26) communiquant, par son autre extrémité, avec l'orifice d'entrée de la poche de recueil (27), la poche de prélèvement (25) étant reliée également, au niveau d'un autre de ses orifices de sortie, à l'une des extrémités d'une tubulure (31) communiquant par son autre extrémité avec l'orifice d'entrée d'un filtre à déleucocyter (32), l'orifice de sortie du filtre à déleucocyter (32) communiquant lui-même par l'intermédiaire d'une tubulure (33) avec une poche de recueil (34).

24. Ensemble de poches (22) selon la revendication 22, **caractérisé en ce qu'**il comporte, en amont de la poche de recueil (27), une aiguille de prélèvement (23) destinée au prélèvement du sang d'un donneur, reliée à l'une des extrémités d'une tubulure (24), elle-même reliée par son autre extrémité à l'orifice d'entrée d'une poche de prélèvement (25), la poche de prélèvement (25) étant elle-même reliée, au niveau de l'un de ses orifices de sortie, à l'une des extrémités d'une tubulure (35) communiquant par son autre extrémité avec l'orifice d'entrée d'un filtre à déleucocyter (32), l'orifice de sortie du filtre (32) étant relié à l'une des extrémités d'une tubulure (36) communiquant par son autre extrémité avec l'orifice d'entrée d'une poche (37), la poche (37) communiquant au niveau de son orifice de sortie avec une tubulure (38) se divisant en deux tubulures (39) et (40) communiquant avec les orifices d'entrée de deux poches (41) et (27) respectivement.

25. Ensemble (22) selon l'une quelconque des revendications 22 à 24, **caractérisé en ce que** les poches ainsi que les tubulures sont souples.

26. Ensemble (22) selon l'une quelconque des revendications 22 à 25, **caractérisé en ce que** les tubulures sont fixes et sécables.

## Claims

1. A filtering bag (1) for retaining the cellular components of plasma by means of filtration, said filtering bag comprising an outer envelope (2, 3) equipped with at least one inlet orifice (8) and at least one outlet orifice (9), the envelope enclosing a filtering medium (11) which delimits two compartments of the filtering bag (1), namely an inlet compartment (6) and an outlet compartment (7), said filtering bag further comprising a pre-filter (10) arranged in the inlet compartment (6) and against the filtering medium (11), said pre-filter (10) comprising at least one layer of a porous material, the pores of the pre-filter (10) being between approximately 8 and approximately 100 microns in size, **characterised in that** the filtering medium (11) of the filtering bag (1) comprises a single layer of a hydrophilic material in the form of a porous membrane of which the pores are sized so as to prevent the passing of cellular components, that is to say are no greater than 3 microns in size.

2. The filtering bag (1) according to claim 1, **characterised in that** the inlet compartment (6) communicates with the environment outside the bag (1) by means of an inlet tube (8).

3. The filtering bag (1) according to claim 1 or 2, **characterised in that** the outlet compartment (7) communicates with the environment outside the bag (1) by means of an outlet tube (9).

4. The filtering bag (1) according to claim 2 or 3, **characterised in that** the inlet tube (8) and outlet tube (9) are flexible.

5. The filtering bag (1) according to any one of claims 1 to 4, **characterised in that** it comprises a post-filter (12) arranged in the outlet compartment (7) of the filtering bag (1) and against the filtering medium (11), the post-filter (12) comprising at least one layer of a porous material, the pores of the post-filter (12) being between approximately 8 and approximately 100 microns in size.

6. The filtering bag (1) according to any one of claims 1 to 5, **characterised in that** the hydrophilic porous material of the filtering medium (11) is selected in particular from natural hydrophilic materials or from materials, in particular plastics-material-based materials, which have been made hydrophilic.

7. The filtering bag (1) according to claim 6, **characterised in that** the porous hydrophilic material is selected in particular from polymers and/or copolymers based on polypropylene, polyester, polyamide, high-density or low-density polyethylene, polyurethane, polyvinylidene fluoride, and cellulose-based products, in particular cellulose acetate and derivatives thereof.

8. The filtering bag (1) according to any one of claims 1 to 7, **characterised in that** the porous material used to produce the post-filter (12) and the pre-filter (10) is selected in particular from plastics-material-based porous materials, in particular polymers and/or copolymers based on high-density or low-density polyethylene, in particular polyethylene terephthalate, and polymers based on polypropylene or polyamide, for example NYLON, for example in the form of a non-woven.

9. The filtering bag (1) according to any one of claims 1 to 8, **characterised in that** the pre-filter (10) and/or the post-filter (12) comprises a plurality of layers of porous material, the different layers being of identical or different nature and having pores of identical or different size.

10. The filtering bag (1) according to any one of claims 1 to 9, **characterised in that** the pre-filter (10) comprises a plurality of porous material layers, the layers of greater porosity being arranged so that the plasma to be filtered entering the inlet compartment (6) first passes through these layers of greater porosity, then through layers of lesser porosity, before reaching the filtering medium (11).

11. The filtering bag (1) according to claim 10, **characterised in that** the pre-filter (10) comprises a layer of polyethylene terephthalate having a mean pore size of approximately 40 microns and arranged on the side of the outer envelope (2), and four layers of polypropylene having a mean pore size of approximately 10 microns and arranged on the side of the filtering medium (11).

12. The filtering bag (1) according to any one of claims 1 to 11, **characterised in that** the filtering medium (11) is more than approximately 10 microns thick, and in particular is approximately 100 microns thick.

13. The filtering bag (1) according to any one of claims 1 to 12, **characterised in that** it has a surface area between approximately 10 cm² and approximately 150 cm², for example 57 cm².

14. The filtering bag (1) according to any one of claims 1 to 13, **characterised in that** the outer envelope (2, 3) is rigid.

15. The filtering bag (1) according to any one of claims 1 to 13, **characterised in that** the outer envelope (2, 3) is flexible.

16. The filtering bag (1) according to claim 15, **characterised in that** the outer envelope is flexible and is formed of two sheets (2, 3) made of flexible plastics material joined over their periphery, the filtering medium (11) and/or the pre-filter (10) and/or the post-filter (12) being held in a flexible and tight framework (5) delimiting, together with the filtering medium (11) and/or the pre-filter (10) and/or the post-filter (12), the inlet compartment (6) and the outlet compartment (7) of the filtering bag (1).

17. The filtering bag (1) according to claim 15 or 16, **characterised in that** the flexible framework (5) is formed of two flexible sheets (13, 14) hemstitched together, between which the filtering medium (11) and/or the pre-filter (10) and/or the post-filter (12) are placed.

18. The filtering bag (1) according to any one of claims 15 to 17, **characterised in that** the sheets (13, 14) forming the flexible framework (5) are fixed together at the periphery of the filtering medium (11) and/or the pre-filter (10) and/or the post-filter (12) and are also fixed to the sheets (2, 3) forming the outer envelope, at the periphery of the outer envelope of the filtering bag (1).

19. The filtering bag (1) according to any one of claims 15 to 18, **characterised in that** the sheets (13, 14) forming the flexible framework (5) are fixed by means of a weld bead (17) formed through the filtering medium (11) and/or the pre-filter (10) and/or the post-filter (12).

20. The filtering bag (1) according to any one of claims 15 to 19, **characterised in that** the outlet compartment (7) is removed from the filtering medium (11) and/or from the post-filter (12) by the presence of one or more spacer rods (20, 21) arranged, inside the outlet compartment (7), between the filtering medium (11) and/or the post-filter (12) and the flexible outer envelope.

21. The filtering bag (1) according to claim 20, **characterised in that** the spacer rod(s) (20, 21) is/are formed from flexible tubes, welded for example at the inner wall of the sheet (3) of the outer envelope.

22. A bag set (22) for separating the cellular components of plasma by means of filtration, said bag set comprising a receiving bag (27) for receiving plasma relieved of some of its cellular components by centrifugation, the receiving bag (27) being connected, at any one of its outlet orifices, to one of the ends of a tube (28) communicating via its other end with the inlet orifice (8) of a filtering bag (1) according to any one of claims 1 to 21, the outlet orifice (9) of the filtering bag (1) being connected to one of the ends of a tube (29), the other end of this tube (29) being connected to a receiving bag (30) for collecting plasma which has been filtered and relived of its cellular components.

23. The bag set (22) according to claim 22, **characterised in that** it comprises, upstream of the receiving bag (27), a sampling needle (23) for taking a sample of blood from a donor and connected to one of the ends of a tube (24), which is in turn connected via its other end to the inlet orifice of a sampling bag (25), the sampling bag (25) in turn being connected at one of its outlet orifices to one of the ends of a tube (26) communicating via its other end with the inlet orifice of the receiving bag (27), the sampling bag (25) also being connected at another of its outlet orifices to one of the ends of a tube (31) communicating via its other end with the inlet orifice of a leukodepletion filter (32), the outlet orifice of the leukodepletion filter (32) in turn communicating by means of a tube (33) with a receiving bag (34).

24. The bag set (22) according to claim 22, **characterised in that** it comprises, upstream of the receiving bag (27), a sampling needle (23) for taking a sample of blood from a donor and connected to one of the ends of a tube (24), which is in turn connected via its other end to the inlet orifice of a sampling bag (25), the sampling bag (25) in turn being connected at one of its outlet orifices to one of the ends of a tube (35) communicating via its other end with the inlet orifice of a leukodepletion filter (32), the outlet orifice of the filter (32) being connected to one of the ends of a tube (36) communicating via its other end with the inlet orifice of a bag (37), the bag (37) communicating at its outlet orifice with a tube (38) dividing into two tubes (39) and (40) communicating with the inlet orifices of two bags (41) and (27) respectively.

25. The set (22) according to any one of claims 22 to 24, **characterised in that** the bags and the tubes are flexible.

26. The set (22) according to any one of claims 22 to 25, **characterised in that** the tubes are fixed and divisible.

## Patentansprüche

1. Filtertasche (1), die dazu bestimmt ist, durch Filtration die Zellbestandteile des Plasmas zurückzuhalten, umfassend eine äußere Hülle (2, 3), die mit mindestens einer Einlassöffnung (8) und mit mindestens einer Auslassöffnung (9) versehen ist, wobei die Hülle ein Filtermedium (11) beinhaltet, das zwei Kammern, Einlasskammer (6) bzw. Auslasskammer (7) der Filtertasche (1) begrenzt und einen Vorfilter (10), der in der Einlasskammer (6) und gegen das Filtermedium (11) angeordnet ist, wobei der Vorfilter (10) mindestens eine Schicht aus einem porösen Material umfasst, wobei die Größe der Poren des Vorfilters (10) im Bereich zwischen etwa 8 und etwa 100 Mikron liegt, **dadurch gekennzeichnet, dass** das Filtermedium (11) der Filtertasche (1) eine einzige Schicht aus einem hydrophilen Material in Form einer porösen Membran umfasst, deren Poren auf eine Größe kalibriert sind, die fähig ist, den Durchgang der Zellbestandteile zu verhindern, nämlich nicht größer als 3 Mikron.

2. Filtertasche (1) nach Anspruch 1, **dadurch gekennzeichnet, dass** die Einlasskammer (6) mit der äußeren Umgebung der Tasche (1) mittels einer Einlassleitung (8) kommuniziert.

3. Filtertasche (1) nach Anspruch 1 oder 2, **dadurch gekennzeichnet, dass** die Auslasskammer (7) mit der äußeren Umgebung der Tasche (1) mittels einer Auslassleitung (9) kommuniziert.

4. Filtertasche (1) nach Anspruch 2 oder 3, **dadurch gekennzeichnet, dass** die Einlassleitung (8) und die Auslassleitung (9) biegsam sind.

5. Filtertasche (1) nach einem der Ansprüche 1 bis 4, **dadurch gekennzeichnet, dass** sie einen Nachfilter (12) umfasst, der in der Auslasskammer (7) der Filtertasche (1) und gegen das Filtermedium (11) angeordnet ist, wobei der Nachfilter (12) mindestens eine Schicht aus einem porösen Material umfasst, wobei die Größe der Poren des Nachfilters (12) im Bereich zwischen etwa 8 und etwa 100 Mikron liegt.

6. Filtertasche (1) nach einem der Ansprüche 1 bis 5, **dadurch gekennzeichnet, dass** das hydrophile poröse Material des Filtermediums (11) insbesondere ausgewählt ist aus den von Natur aus hydrophilen Materialien oder den Materialien, insbesondere auf Basis von Kunststoff, die hydrophil gemacht wurden.

7. Filtertasche (1) nach Anspruch 6, **dadurch gekennzeichnet, dass** das poröse hydrophile Material insbesondere ausgewählt ist aus den Polymeren und/oder Copolymeren auf der Basis von Polypropylen, Polyester, Polyamid, Polyethylen hoher oder niedriger Dichte, Polyurethan, Polyvinylidenfluorid, den Produkten auf der Basis von Cellulose, insbesondere Celluloseacetat und dessen Derivaten.

8. Filtertasche (1) nach einem der Ansprüche 1 bis 7, **dadurch gekennzeichnet, dass** das poröse Material, das für die Ausführung des Nachfilters (12) und des Vorfilters (10) verwendet wird, insbesondere aus den porösen Materialien auf der Basis von Kunststoffmaterial ausgewählt wird, insbesondere den Polymeren und/oder Copolymeren auf der Basis von Polyethylen hoher oder niedriger Dichte, insbesondere Polyethylenterephtalat, den Polymeren auf der Basis von Polypropylen, Polyamid, zum Beispiel NYLON, zum Beispiel in Form eines Vlieses.

9. Filtertasche (1) nach einem der Ansprüche 1 bis 8, **dadurch gekennzeichnet, dass** der Vorfilter (10) und/oder der Nachfilter (12) mehrere gleichartige oder verschiedene Schichten aus porösem Material mit gleicher oder verschiedener Porengröße umfasst.

10. Filtertasche (1) nach einem der Ansprüche 1 bis 9, **dadurch gekennzeichnet, dass** der Vorfilter (10) mehrere Schichten aus porösem Material umfasst, wobei die Schichten mit der größten Porosität derart angeordnet sind, dass das zu filtrierende Plasma, das in die Einlasskammer (6) eintritt, zuerst diese Schichten mit der größten Porosität, dann anschließend die Schichten mit geringerer Porosität durchläuft, bevor es das Filtermedium (11) erreicht.

11. Filtertasche (1) nach Anspruch 10, **dadurch gekennzeichnet, dass** der Vorfilter (10) eine Polyethylenterephtalatschicht mit einer mittleren Porengröße von etwa 40 Mikron, die auf der Seite der äußeren Hülle (2) angeordnet ist und vier Polypropylenschichten mit einer mittleren Porengröße von etwa 10 Mikron, die auf der Seite des Filtermediums (11) angeordnet sind, umfasst.

12. Filtertasche (1) nach einem der Ansprüche 1 bis 11, **dadurch gekennzeichnet, dass** das Filtermedium (11) eine Dicke von mehr als etwa 10 Mikron, insbesondere von etwa 100 Mikron, aufweist.

13. Filtertasche (1) nach einem der Ansprüche 1 bis 12, **dadurch gekennzeichnet, dass** sie eine Oberfläche im Bereich zwischen etwa 10 cm² und etwa 150 cm², zum Beispiel 57 cm², aufweist

14. Filtertasche (1) nach einem der Ansprüche 1 bis 13, **dadurch gekennzeichnet, dass** die äußere Hülle (2, 3) starr ist.

15. Filtertasche (1) nach einem der Ansprüche 1 bis 13, **dadurch gekennzeichnet, dass** die äußere Hülle (2, 3) biegsam ist.

16. Filtertasche (1) nach Anspruch 15, **dadurch gekennzeichnet, dass** die biegsame äußere Hülle aus zwei Platten (2, 3) aus biegsamem Kunststoffmaterial gebildet ist, die an ihrem Rand zusammengefügt wurden, wobei das Filtermedium (11) und/oder der Vorfilter (10) und/oder der Nachfilter (12) in einem biegsamen und dichten Rahmen (5) gehalten werden, der mit dem Filtermedium (11) und/oder dem Vorfilter (10) und/oder dem Nachfilter (12) die Einlasskammer (6) und die Auslasskammer (7) der Filtertasche (1) begrenzt.

17. Filtertasche (1) nach Anspruch 15 oder 16, **dadurch gekennzeichnet, dass** der biegsame Rahmen (5) aus zwei biegsamen Platten (13, 14), die gegeneinander durchbrochen sind, gebildet ist, zwischen denen das Filtermedium (11) und/oder der Vorfilter (10) und/oder der Nachfilter (12) angebracht sind.

18. Filtertasche (1) nach einem der Ansprüche 15 bis 17, **dadurch gekennzeichnet, dass** die Platten (13, 14), die den biegsamen Rahmen (5) bilden, aneinander in Höhe des Rands des Filtermediums (11) und/oder des Vorfilters (10) und/oder des Nachfilters (12) und auch an den Platten (2, 3), die die äußere Hülle des äußeren Rands der Filtertasche (1) bilden, in Höhe des Rands befestigt sind.

19. Filtertasche (1) nach einem der Ansprüche 15 bis 18, **dadurch gekennzeichnet, dass** die Befestigung der Platten (13, 14), die den biegsamen Rahmen (5) bilden, eine Schweißnaht (17) ist, die durch das Filtermedium (11) und/oder den Vorfilter (10) und/oder den Nachfilter (12) ausgeführt ist.

20. Filtertasche (1) nach einem der Ansprüche 15 bis 19, **dadurch gekennzeichnet, dass** die Auslasskammer (7) vom Filtermedium (11) und/oder dem Nachfilter (12) durch die Gegenwart eines oder mehrerer Abstandshalter (20, 21) abgesetzt ist, die zwischen dem Filtermedium (11) und/oder dem Nachfilter (12) und der biegsamen äußeren Hülle, im Innern der Auslasskammer (7) angeordnet sind,

21. Filtertasche (1) nach Anspruch 20, **dadurch gekennzeichnet, dass** der oder die Abstandshalter (20, 21) aus biegsamen Rohren ausgeführt sind, die zum Beispiel in Höhe der Innenwand der Platte (3) der äußeren Hülle angeschweißt sind.

22. Taschensystem (22) zur Trennung der Zellbestandteile des Plasmas durch Filtration, umfassend eine Sammeltasche (27), die das durch Zentrifugation von seinen Zellbestandteilen befreite Plasma aufnimmt, wobei die Sammeltasche (27) in Höhe einer ihrer Auslassöffnungen mit einem der Enden einer Leitung (28) verbunden ist, wobei sie über ihr anderes Ende mit der Einlassöffnung (8) einer Filtertasche (1) nach einem der Ansprüche 1 bis 21 kommuniziert, wobei die Auslassöffnung (9) der Filtertasche (1) mit einem der Enden einer Leitung (29) verbunden ist, wobei das andere Ende dieser Leitung (29) mit einer Sammeltasche (30) verbunden ist, die dazu bestimmt ist, das filtrierte und von seinen Zellbestandteilen befreite Plasma zu sammeln.

23. Taschensystem (22) nach Anspruch 22, **dadurch gekennzeichnet, dass** es stromaufwärts der Sammeltasche (27) eine Entnahmenadel (23) umfasst, die zur Entnahme des Bluts eines Spenders bestimmt ist, die mit einem der Enden einer Leitung (24) verbunden ist, die ihrerseits über ihr anderes Ende mit der Einlassöffnung einer Entnahmetasche (25) verbunden ist, wobei die Entnahmetasche (25) ihrerseits in Höhe einer ihrer Auslassöffnungen mit einem der Enden einer Leitung (26) verbunden ist, die über ihr anderes Ende mit der Einlassöffnung der Sammeltasche (27) kommuniziert, wobei die Entnahmetasche (25) ebenfalls in Höhe einer ihrer anderen Auslassöffnungen mit einem der Enden einer Leitung (31) verbunden ist, die über ihr anderes Ende mit der Einlassöffnung eines Filters zur Leukozytenentfernung (32) kommuniziert, wobei die Auslassöffnung des Filter zur Leukozytenentfernung (32) ihrerseits mittels einer Leitung (33) mit einer Sammeltasche (34) kommuniziert.

24. Taschensystem (22) nach Anspruch 22, **dadurch gekennzeichnet, dass** es stromaufwärts der Sammeltasche (27) eine Entnahmenadel (23) umfasst, die zur Entnahme des Bluts eines Spenders bestimmt ist, die mit einem der Enden einer Leitung (24) verbunden ist, die ihrerseits über ihr anderes Ende mit der Einlassöffnung einer Entnahmetasche (25) verbunden ist, wobei die Entnahmetasche (25) ihrerseits in Höhe einer ihrer Auslassöffnungen mit einem der Enden einer Leitung (35) verbunden ist, die über ihr anderes Ende mit der Einlassöffnung eines Filters zur Leukozytenentfernung (32) kommuniziert, wobei die Auslassöffnung des Filters (32) mit einem der Enden einer Leitung (36) verbunden ist, die über ihrem anderen Ende mit der Einlassöffnung einer Tasche (37) kommuniziert, wobei die Tasche (37) in Höhe ihrer Auslassöffnung mit einer Leitung (38) kommuniziert, die sich in zwei Leitungen (39) und (40) teilt, die jeweils mit der Einlassöffnung der beiden Taschen (41) bzw. (27) kommuniziert.

25. System (22) nach einem der Ansprüche 22 bis 24, **dadurch gekennzeichnet, dass** die Taschen sowie die Leitungen biegsam sind.

26. System (22) nach einem der Ansprüche 22 bis 25, **dadurch gekennzeichnet, dass** die Leitungen befestigt und teilbar sind.
